# EUROPEAN PATENT APPLICATION

(11) **EP 4 427 750 A1**
(43) Date of publication of application: **11.09.2024**
(21) Application number: 23160777.1
(22) Date of filing: 08.03.2023
(51) Int. Cl.: A61K 31/506, A61P 35/00, A61K 31/136, A61K 31/585

(54) **TREATMENT OF LUNG ADENOCARCINOMA**

(71) Applicant: Bash Biotech Inc, San Diego, CA 92101 (US)
(72) Inventor: MARDINOGLU, Adil, 412 60 Göteborg (SE); LI, Xiangyu, 113 46 Stockholm (SE)
(74) Representative: Kransell & Wennborg KB

(57) **Abstract**

The present disclosure provides CGP-60474 for use in a method of prevention or treatment of lung adenocarcinoma and mitoxantrone for use in a method of treatment of stage o or I lung adenocarcinoma.

## Description

### TECHNICAL FIELD

The present disclosure relates to the field of treatment of adenocarcinoma.

### BACKGROUND

Lung cancer is the most lethal cancer worldwide, causing more than 1.7 million deaths in 2020¹. Lung adenocarcinoma (LUAD) is the most prevalent histologic subtype in lung cancer, and accounts for half of all lung cancer deaths ². LUAD has a strong association with smoking history, while it also showed an increasing occurrence in non-smokers ². Patients with localized and early-stage tumor receive standard surgical resection, but the majority of patients are usually diagnosed at an advanced stage and recommended the conventional therapies including chemoradiotherapy, targeted therapy and immunotherapy. Various mutations in genes *EGFR, ALK, KRAS, ROS1, BRAF, NTRK1*/*2*/*3, MET, RET,* and *ERBB2* are observed in LUAD patients with relatively high coverages and these genes are involved in the molecular testing for patients' targeted therapy recommendation in clinical practice ³. For instance, the first-line treatment for patients with *EGFR* activating mutations includes tyrosine kinase inhibitors osimertinib, erlotinib, afatinib, gefitinib and dacomitinib. Despite these different therapy regimens, 5-year overall survival rate ranges from 4 to 17% depending on different tumor stages and regions ⁴. Thus, there is need to discover new drug targets and develop effective drugs for LUAD treatment, and thus improve the survival outcomes of patients.

Numerous studies have focused on the identification of biomarker genes based on the bioinformatics analysis of transcriptomics profiles of LUAD patients ⁵⁻¹³. These studies validated the importance of the identified biomarkers by proving the prognostic value of these genes or linking these genes with some meaningful tumor hallmarks, e.g., immune infiltration, cell differentiation or glycolysis. However, none of these studies attempted to find the potential drug candidates for targeting the marker genes. Computational drug repositioning (CDR), aiming to identify new uses of existing drugs based on data driven analysis, has now been widely used in drug discovery for different human diseases. Traditional drug discovery utilizes a *de novo* design approach, which requires high cost and many years of drug development before it reaches the market. In contrast, CDR can dramatically decrease the overall cost, time duration and development risks of bringing the drug to market because it aims to reuse the clinical drugs or compounds under medical investigation, and the safety, pharmacokinetic profiles, formulation development and even bulk manufacturing of these drugs have been well-characterized in early medical investigation and these processes can be therefore by-passed. The most meaningful examples were observed from the recent outbreak of Covid-19. JAK inhibitor baricitinib and antiviral medicine paxlovid (nirmatrelvir /ritonavir) have been authorized for emergency use by the FDA for the treatment of Covid-19 patients. Baricitinib and paxlovid were previously used for treatment of rheumatoid arthritis and HIV infection, respectively.

CDR, including various data-driven approaches such as drug/protein structure-based similarity analysis, gene expression profile-based signature matching or pathway modulating, provides a systematic way to discover all possible pairs between existed drugs and disease targets or pathways¹⁴⁻¹⁷. It is also helpful to explain the mechanisms of action of drugs for disease treatment. Especially, profile-based CDR does not rely on the prior knowledge, and it increases the ability to discovery new drug-disease-target pairs ¹⁸. It has been reported that drug repositioning accounts for approximately 30% of the newly FDA-approved drugs and vaccines in recent years ^{19,20}. Huang et al. identified 1,597 LUAD-related genes and applied a network-overlap and -distance based drug repositioning method to detect the potential drug candidates for targeting these genes ²¹. However, this study did not provide any experimental validation for the drug efficacy. Bastiani et al. ²² and Kwon et al. ²³ employed a profile-based drug repositioning approach by mapping the drug-induced and LUAD-associated gene expression profiles based on the hypothesis that a drug is considered to have a therapeutic worth if it can reverse the disease-associated molecular dysregulation. However, a drug identified by this method is supposed to have multiple gene targets, mixed with driver oncogenes and passenger genes, which limits the understanding and validation of the drug mechanism of action.

### SUMMARY

To solve the above-mentioned problem, the present inventors recently proposed a computational drug repositioning method to predict the drugs that can target a specific gene instead of general regulation of a set of disease-related genes ¹⁵. Moreover, the present inventors have validated the feasibility of the drug repositioning approach for the treatment of kidney and liver cancers as well as non-alcoholic fatty liver disease ^{15,24,25}.

In the study presented herein, the hub genes that drives the LUAD development were identified and promising drugs that can target these genes were repurposed. First, the consensus prognostic genes were identified in two independent LUAD cohorts based on survival analysis. Secondly, cohort-specific gene co-expression networks were constructed based on their transcriptomics profiles and a set of gene modules associated with the patients' prognoses was identified. Further, potentially promising drugs for these targets were identified and the drug efficacy was validated by performing *in vitro* cell line experiments.

This study demonstrates that the inventors' method is feasible in the therapeutic target identification and drug repositioning in LUAD. Nine targetable genes (*KIF23, CDCA8, MAD2L1, MCM6, TPX2, NCAPH, CLSPN, TTK,* and *KIF4A*) that are associated with patients' survival outcomes were identified. According to the study results, these genes play a central role in key functional modules and are also essential for tumor cell growth. Moreover, one clinically used drug (i.e. mitoxantrone) and two drugs under medical investigation (i.e. CGP-60474 and wortmannin) that target *MCM6* or *TTK* were identified for the treatment of LUAD patients.

Two phase II studies have failed to validate the antitumor activity of mitoxantrone in the treatment for lung cancer ^{50,51}. However, both studies had small sample sizes (each recruited 24 patients), and only advanced or metastatic non-small-cell lung cancer were involved. Based on the analysis of the present inventors, mitoxantrone should be used for the treatment of LUAD patients with early-stage tumors, since its target gene *MCM6* is particularly powerful for the classification of stage I tumors ^{5.6}.

In the prior art, CGP-60474 (CAS No.: 164658-13-3) is described as a highly potent anti-endotoxemic agent and a potent cyclin-dependent kinase (CDK) inhibitor that is also a selective and ATP-competitive PKC inhibitor. It has not been connected to lung cancer in the prior art.

Wortmannin is a fungal metabolite that is identified as a potent and selective inhibitor for phosphoinositide 3-kinases (PI3Ks) ⁵². It has been reported that wortmannin inhibits the growth of non-small cell lung cancer in *in vitro* and in vivo models ⁵³ and its treatment reverses the cisplatin resistance in lung cancer cells ⁵⁴. Wortmannin has thus been connected to lung cancer in the prior art.

Accordingly, the present disclosure provides CGP-60474 for use in a method of prevention or treatment of lung adenocarcinoma and mitoxantrone for use in a method of treatment of stage 0 or I lung adenocarcinoma.

### BRIEF DESCRIPTION OF THE FIGURES

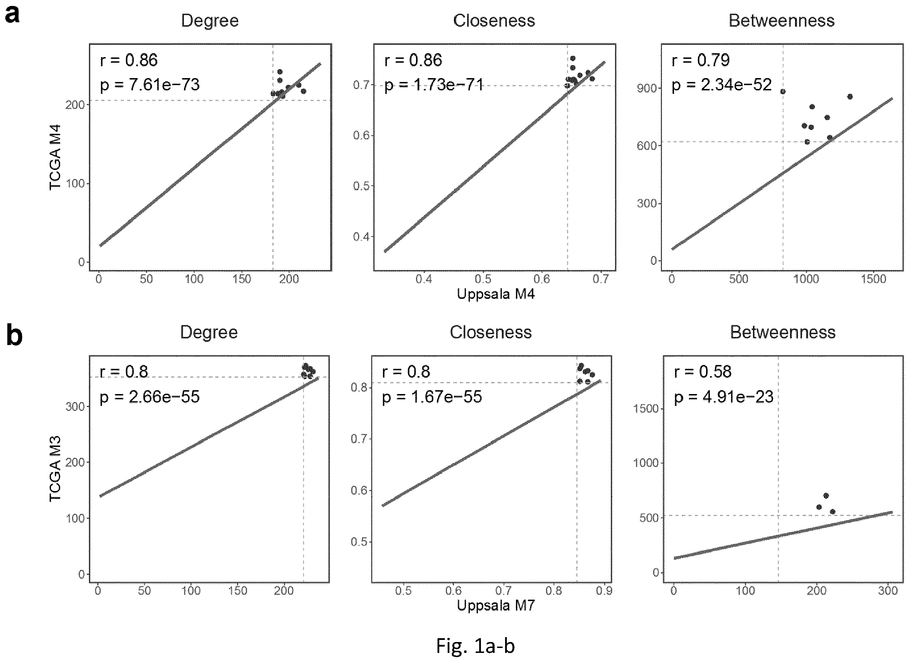

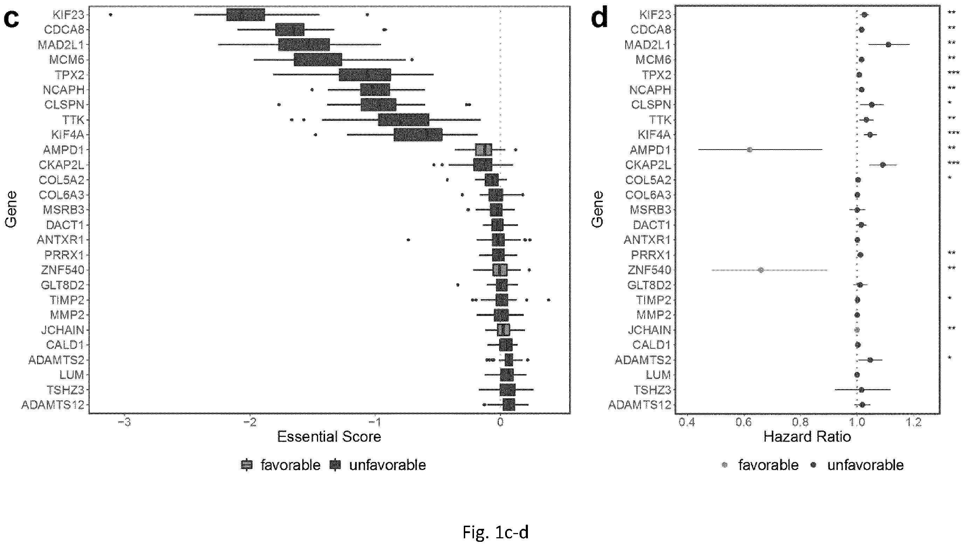

Figure 1. Identification of the targetable genes. Spearman correlation of centrality measurements (a) between TCGA M4 and Uppsala M4, and (b) between TCGA M3 and Uppsala M7. Each dot (black) represents a gene that is ranked within the top 20 based on a descending order of the corresponding centrality measurement. The diagonal lines show a linear fit with the shaded region showing the 95% confidence intervals. In addition, r and p represent the Spearman correlation coefficient and p-value, respectively. (c) Essential scores of the selected genes in 50 LUAD cell lines. (d) Hazard ratios of the selected genes. Lines indicate 95% confidence intervals. Significance levels: * p < 0.05, ** p < 0.01, *** p < 0.001.
Figure Drug repositioning and validation of drug efficacy in an *in vitro* model. (a) Correlation coefficients of the top drugs in A549 and HCC515 cell lines. The drug candidates were ranked by the mean values of correlation coefficients from the two cell lines. (b) Bar plot showing the cell viability of A549 cell line after drug treatment in MTT assays. (c) Bar plot showing the cytotoxicity on A549 cell lines after drug treatment in LDH assays. "Positive" and "negative" represent the lysis cells and DMSO treated cells, respectively. Difference between groups was estimated by the Student's t-test. Western blots showing the inhibitory effect of drugs on the protein expression of (d) *MCM6* and (e) *TTK*. Error bar represents standard error. Significance levels: * p< 0.05, ** p< 0.01, *** p< 0.001. 10µM CGP-60474, 10µM wortmannin and 100nM mitoxantrone were used for cell treatment.
Figure 3. Molecular docking analysis of predicted drugs and their corresponding targets. (a) Putative binding modes of mitoxantrone, CGP-60474, and wortmannin against *TTK*. (b) Putative binding modes of mitoxantrone and CGP-60474 against *MCM6.* Hydrogen bonds are presented as dashed lines.
Figure 4. Cell viability of A549 cell line after drug treatment with different concentrations.
Figure 5. Raw images of Western blots showing the inhibitory effect of drugs on the protein expression levels of *MCM6* and *TTK.*

### DETAILED DESCRIPTION

As described herein, an integrated approach has been employed to identify therapeutic targets and previously known drugs have been repurposed for LUAD treatment. The treatment may help patients who cannot benefit from the chemotherapies or targeted therapies that are used in the clinic today. The identified druggable gene targets are *CDCA8, MCM6* and *TTK.*

*CDCA8* encodes a component of the chromosomal passenger complex which is an essential regulator for mitosis and cell division. It has been reported that the phosphorylation and activation of *CDCA8* plays a key role in lung carcinogenesis and the suppression of *CDCA8* significantly inhibits the growth of lung cancer cells ⁴³, which supports the findings presented herein.

*MCM6* encodes one of the highly conserved mini-chromosome maintenance proteins that are essential for the imitation of eukaryotic genome replication. *MCM6* is involved in the prognostic signatures for indicating the survival outcomes for particularly early-stage LUAD patients ^{5,6}.

*TTK* encodes a dual specificity protein kinase with the ability to phosphorylate tyrosine, serine and threonine, which is a critical mitotic checkpoint protein for accurate segregation of chromosomes during mitosis ⁴⁴. It has been reported that *TTK* is overexpressed in tumor tissues compared to normal lung tissue and its selective inhibitor CFI-402257 shows significant antineoplastic activity in LUAD mouse models ⁴⁵.

Further, the present inventors predicted that oxetane, mitoxantrone, staurosporine, CGP-60474 and alvocidib had a potential inhibitory effect on the expression of *MCM6,* and that mitoxantrone, wortmannin, and NVP-BEZ235 had a potential inhibitory effect on the expression of *TTK.* Among these drugs, the drug efficacy of mitoxantrone and CGP-60474 for targeting *MCM6,* and mitoxantrone and wortmannin for targeting *TTK* was validated in an *in vitro* model. Interestingly, CGP-60474 also suppressed the protein level of *TTK*. In the drug repositioning analysis, the present inventors set that the correlation coefficient in both lung cancer cell line A549 and HCC515 should be higher than 0.4 based on previously empirical setting in kidney cancer and liver diseases ^{15,24,25}. However, it was observed that the correlation coefficient of interaction between CGP-60474 and *TTK* was 0.3895 in the cell line A549, which was in border line. Thus, unfortunately, this interaction was skipped based on above criterion during the prediction. CGP-60474 is a potent inhibitor of *CDK1* which is one of the cyclin-dependent kinases ⁴⁶. Both *CDK1* and *TTK* are involved the control of G1/S and G2/M phases ^{47,48} and these two genes showed a high correlation in expression across patients from LUAD TCGA cohort in the analysis (Spearman correlation coefficient = 0.88), which might explain that CGP-60474 can target *TTK.* Mitoxantrone is an inhibitor of DNA topoisomerase II alpha (*TOP2A*), and it leads to cell deaths by the induction of double stranded DNA breaks ⁴⁹. It is currently applied in the treatment of breast and prostate cancers, lymphomas and leukemias. There were two phase II studies that failed to validate the antitumor activity of mitoxantrone in the treatment for lung cancer ^{50,51}. Both studies had small sample sizes (each recruited 24 patients), and only advanced or metastatic non-small-cell lung cancer were involved. Based on the present inventors' analysis, mitoxantrone should however be used for the treatment of LUAD patients with early-stage tumors, since its target gene *MCM6* is particularly powerful for the classification of stage I tumors 5,6.

In the study presented herein, two different LUAD cohorts in which the patients had different geographic characteristics and cultural backgrounds were used. Despite these heterogeneities, repeatable prognostic genes, functional gene modules and druggable targets were found.

As a first aspect of the present disclosure, there is provided CGP-60474 or a pharmaceutically acceptable salt thereof for use in a method of prevention or treatment of lung adenocarcinoma.

As shown herein, CGP-60474 modulates *MCM6.* The present inventors foresee that the preventive or therapeutic effect may be enhanced by simultaneous, sequential or separate administration of an agent modulating *TTK.* Accordingly, in an embodiment of the first aspect, the method further comprises administration of a drug selected from the group consisting of mitoxantrone, wortmannin and NVP-BEZ235.

The present inventors also foresee that the preventive or therapeutic effect may be enhanced by simultaneous, sequential or separate administration of an agent modulating *CDCA8.* Accordingly, in an alternative or complimentary embodiment of the first aspect, the method further comprises administration of a drug selected from the group consisting of BRD-K43256821, EMF-BCA1-57, GSK-1059615, curcumin, and alvocidib.

In an embodiment of the first aspect, said lung adenocarcinoma expresses *MCM6.* As an example, said lung adenocarcinoma may overexpress *MCM6* as compared to an average expression value obtained from measurements in healthy tissue.

Accordingly, the method of the first aspect may comprise detecting *MCM6* expression in said lung adenocarcinoma and administration of CGP-60474 or a pharmaceutically acceptable salt thereof to treat said lung adenocarcinoma subsequent to detection of *MCM6* expression.

Further, the method of the first aspect may comprise the steps of:
i) measuring the degree of *MCM6* expression in said lung adenocarcinoma to obtain a *MCM6* expression value;
ii) comparing the *MCM6* expression value to a reference value, e.g. an average value obtained from measurements in healthy tissue; and
iii) administration of CGP-60474 or a pharmaceutically acceptable salt thereof to treat said lung adenocarcinoma if the *MCM6* expression value is higher than the reference value.

The person skilled in the art of lung adenocarcinomas and treatment thereof is capable of establishing a relevant reference value, e.g., by selecting relevant samples of healthy lung tissue and making measurements of the *MCM6* expression therein.

As a second aspect of the present disclosure, there is provided mitoxantrone or a pharmaceutically acceptable salt thereof for use in a method of treatment of stage 0 or I lung adenocarcinoma.

In said method, mitoxantrone is preferably administered by injection, e.g. intravenous injection.

As shown herein, mitoxantrone modulates *TTK* and *MCM6.* The present inventors foresee that the preventive or therapeutic effect may be enhanced by simultaneous, sequential or separate administration of an agent targeting *CDCA8.* Accordingly, in an embodiment of the second aspect, the method further comprises administration of a drug selected from the group consisting of BRD-K43256821, EMF-BCA1-57, GSK-1059615, curcumin, and alvocidib.

In an embodiment of the second aspect, said lung adenocarcinoma expresses *TTK.* As an example, said lung adenocarcinoma may overexpress *TTK* as compared to an average expression value obtained from measurements in healthy tissue.

Accordingly, the method of the second aspect may comprise detecting *TTK* expression in said lung adenocarcinoma and administration of mitoxantrone or a pharmaceutically acceptable salt thereof to treat said lung adenocarcinoma subsequent to detection of *TTK* expression.

Further, the method of the second aspect may comprise the steps of:
i) measuring the degree of *TTK* expression in said lung adenocarcinoma to obtain a *TTK* expression value;
ii) comparing the *TTK* expression value to a reference value, e.g. an average value obtained from measurements in healthy tissue; and
iii) administration of mitoxantrone or a pharmaceutically acceptable salt thereof to treat said lung adenocarcinoma if the *TTK* expression value is higher than the reference value.

The person skilled in the art of lung adenocarcinomas and treatment thereof is capable of establishing a relevant reference value, e.g. by selecting relevant samples of healthy lung tissue and making measurements of the *TTK* expression therein.

As a third aspect of the present disclosure, there is provided a method of prevention or treatment of lung adenocarcinoma comprising administration of CGP-60474 or a pharmaceutically acceptable salt thereof to a subject in need thereof.

For the reasons set out above, the method of the third aspect may further comprise administration of:
a drug selected from the group consisting of mitoxantrone, wortmannin and NVP-BEZ235; and/or
a drug selected from the group consisting of BRD-K43256821, EMF-BCA1-57, GSK-1059615, curcumin, and alvocidib.

As a fourth aspect of the present disclosure, there is provided a method of treatment of stage 0 or I lung adenocarcinoma comprising administration of mitoxantrone or a pharmaceutically acceptable salt thereof to a subject in need thereof.

For the reasons set out above, the method of the fourth aspect may further comprise administration of:
a drug selected from the group consisting of oxetane, staurosporine, CGP-60474 and alvocidib; and/or
a drug selected from the group consisting of BRD-K43256821, EMF-BCA1-57, GSK-1059615, curcumin, and alvocidib.

The lung adenocarcinoma stage referred to above is the AJCC stage, which is preferably determined according to the eighth edition of lung cancer stage classification (based on the TNM system).

The other embodiments of the first and second aspect described above apply *mutatis mutandis* to the third and fourth aspect, respectively.

The subject of the above aspects is preferably a human.

### EXAMPLES

### Methods

To identify the therapeutic target genes and effective drugs for targeting these targets, four steps of systems biology-based analyses based on the RNA-seq profiles of two independent LUAD cohorts were performed:
Step 1. Identify the prognostic genes by survival analysis;
Step Find the central prognostic genes based on the gene co-expression network analysis;
Step 3. Rank the drugs based on a profile-based drug repositioning approach; and
Step 4. Validate the drug efficacy in *in vitro* model.

The methodology is described in more detail as below.

### Data Pre-processing

The metadata and raw RNA-seq data of 497 LUAD patients from the TCGA cohort were downloaded from the GDC data portal (https://portal.gdc.cancer.gov/) ²⁶. Another independent cohort is from 199 non-small cell lung cancer patients surgically treated during 2006-2010 at the Uppsala University Hospital, Uppsala, Sweden (NCBI SRA database: SRP074349) ^{27,28}. From this cohort, only the RNA-seq data of the 105 LUAD patients whose survival information was available was used. For both datasets, count and transcripts per million (TPM) values of transcripts were quantified by Kallisto based on the human reference genome Ensembl V103 ²⁹. Only protein-coding transcripts were filtered for further analysis. The sum count or TPM value of different alternative splicing transcripts from a gene was used as the gene expression value of this gene. Moreover, the genes with average TPM values >1 were analyzed in this study.

### Survival Analysis

Cox proportional hazard regression was used to evaluate the association between gene expression levels and patients' overall survival (OS) based on the 'coxph' function from the R package 'survival' ³⁰. P-value < 0.01 was used to identify the significantly prognostic genes.

### Functional Enrichment Analysis

The `enrichGO' function from the R package 'clusterProfiler' (version 4.2.2) was used for the Gene ontology (GO) enrichment analysis, which detected the significantly enriched GO terms based on the hypergeometric distribution ³¹. The 'org.Hs.eg.db' database was used as the GO term source (version 3.14.0). GO terms with FDR < 0.05 were used for further analysis. P-values were adjusted based on the Benjamini-Hochberg method.

### Gene Co-expression Network Analysis

Based on the TPM values of genes, the Spearman correlation was employed to calculate the association of all possible gene pairs across all tumor samples in each cohort. Then, the gene co-expression network was constructed by extracting the gene-to-gene links ranked within top 1% based on the highest correlation coefficients in each cohort. Then, a random walks-based algorithm, named Walktrap, was used to identify the gene modules with high transitivity ³². The function 'cluster_walktrap' in R package `igraph' was used to implement the Walktrap algorithm. Unweighted edges were used for community detection (weights=NULL) and the default settings recommended by this function were used for other parameters. Modules with more than 50 genes and clustering coefficients higher than 0.5 were used for further analysis. Clustering coefficient ranges from 0 and 1, and value higher than 0.5 is a commonly used cutoff which indicates that the partitions have a good cluster tendency and the genes involved in each module show a proper connectivity 33. Degree, closeness, and betweenness values of genes were calculated in each module based on the R package `igraph'.

### Concordance Analysis

The accumulative hypergeometric model was used to determine whether two lists of prognostic genes had a significant overlap as previously described ¹⁵. This model was also used to test to determine whether the genes involved in a module had a significant overlap with the prognostic genes. Jaccard indices were calculated as the size of the overlapped genes divided by the size of the union of two lists of genes.

### Essential Scores of Genes

The essential scores of genes in 50 LUAD cell lines were obtained from the DepMap Portal (https://depmap.org/portal/), which were calculated based on the CRISPR-Cas9 essentiality screens while accounting for the copy number-specific effect ³⁴. The score of a gene indicates the essentiality of this gene for cancer cell proliferation and survival after knockout of this gene by CRISPR-Cas9 technology. A more negative score indicates that the gene is more essential.

### Drug Repositioning for Target Genes

The previously proposed drug repositioning approach was applied to identify drugs that can inhibit the target genes ^{15,24}. This method hypothesizes that a drug is considered to have an inhibitory effect on the expression of a target gene if this drug leads to a wide perturbation on the gene expression landscape in tumor cells which is similar to the effect of the knockdown of this target gene. The shRNA gene knockdown and drug perturbed transcriptomic gene signature profiles (level 5) of two LUAD cell lines A549 and HCC515 were downloaded from the Expanded CMap LINCS Resource (version 2020, https://clue.io/data/CMap2020#LINCS2020). The level 5 data provides the replicate-collapsed Z-scores, representing a consensus biological response of transcriptomics to the perturbation of drug treatment or gene knockdown derived from different replicates ³⁵. The drug repositioning analysis was performed for *CDCA8, MCM6,* and *TTK* since these genes had the available gene knockdown transcriptomic signature profiles. Each gene was knocked down by three different shRNAs, thus three gene knockdown perturbed signature profiles for each gene in both A549 and HCC515 cell lines were obtained. Meanwhile, 53,827 drug-perturbed signature profiles associated with 13,945 unique drugs with different dosages and treatment durations in A549 cell line and 16,133 drug-perturbed signature profiles associated with 5,890 unique drugs in HCC515 cell line were obtained.

In brief, there were four steps in the drug repositioning analysis. First, for a given gene, a drug-shRNA matrix was created by computing the Spearman correlation between this gene knockdown and drug treatment perturbed signature profiles in each cell line. In this matrix, each row represents a drug perturbagen with a specific dosage and treatment duration, and each column represents a gene knockdown with a specific shRNA. Second, the drug-shRNA matrix was simplified by selecting the optimal drug and shRNA perturbagens. A drug was added to the cells with different dosages and treatment durations in the CMap experimental setting. To maximize the drug effect, only a unique and optimal dosage/duration that showed the highest correlation coefficients with each shRNA perturbagen was kept for each drug. Similarly, a gene was knocked down by three different shRNAs. To maximize the gene knockdown effect, only the optimal shRNA perturbagen for each gene was kept. We calculated the mean correlation coefficient of each shRNA perturbagen based on its correlation with all drugs' perturbagens. Then, the shRNA perturbagen with highest mean correlation coefficient is optimal. After optimization, each coefficient in the drug-shRNA matrix represents the similarity between the effects induced by a specific drug treatment (row) and a specific gene knockdown (column). Higher coefficient value means high possibility of the inhibitory effect of a drug on its target gene. Third, the drug-shRNA matrices from the two cell lines were merged by extracting the common drugs. Drugs that were not presented in both cell lines were excluded. Finally, for each gene, the top five drugs with the highest mean coefficients from the two cell lines were selected as the most effective drugs. Meanwhile, the coefficients in both cell lines should be greater than 0.4 which was an empirical cutoff based on a previous *in vitro* experimental validation ^{15,25}.

### Cell culture, MTT, and LDH assay

A549 cells were obtained from ATCC and cultured with culture medium (D0819 DMEM 10% FBS 1% penicillin/streptomycin) at 37°C. Cell viability was measured using an MTT assay. MTT ((3-[4,5-dimethylthiazol-2-yl]-2,5-diphenyltetrazolium bromide) (1x), at 5 mg/ml) was added for 1 hour and formazan was dissolved with DMSO (dimethyl sulfoxide). Optical density at a wavelength of 570 nm was measured using a microplate reader (Hidex plate reader) to quantify the relative number of living cells. To determine treatment concentrations, cells were treated with serially diluted drugs until a concentration that reduced cell viability by around 50%. As a result, 10µM for CGP-60474 (MedChemExpress, HY-11009), 10µM for wortmannin (Sigma, W1628) and 100nM mitoxantrone (Selleckchem, NSC-301739) were used. Cells were treated with the drugs at their designated concentrations for two days.

Cytotoxic effect on the cells was measured with lactate dehydrogenase (LDH) assays that were performed in triplicates at the same concentrations as selected for the MTT assays following the LDH kit procedure (Abeam Cat# ab65393). 30 minutes after LDH (Abcam Cat# ab65393) administration, optical density at a wavelength of 450 nm was measured using a microplate reader (Hidex plate reader) to quantify the relative LDH activity which was released into the media via the drug's cytotoxic effect.

To normalize the quantification results, the mean negative control (untreated cells) reading was subtracted from each reading and then each was divided by the mean positive control (Cell Lysis Solution) reading minus the mean negative control reading.

### Western Blotting

Triplicates of drug treatments at the same concentrations as selected for the MTT assays were prepared for the Western blots. Whole cell lysate was extracted with CelLytic M (C2978, Sigma-Aldrich) lysis buffer. The protein content of each sample was measured using a Bradford assay and each sample was prepared for Western blotting using 2× Laemmli buffer. Electrophoresis was run at 150 V for 40 minutes and membrane transfer was performed with the Trans-Blot Turbo Transfer System (Biorad). PVDF membranes were blocked for 30 minutes with 5% skim milk and TBST (Tris Buffered Saline with 0.2% Tween 20) and then treated overnight on a shaker plate at 4°C with 0.5% skim milk with TBST and *MCM6* (Abcam Cat# ab201683) and *TTK* (Abcam Cat# ab187520) primary antibodies diluted 1:20000 and for 30 minutes with *TUBA1A* (Abeam Cat# ab7291, RRID:AB_2241126) primary antibodies diluted 1:20000. The solution was then replaced with a new solution of 0.5% skim milk with TBST and anti-rabbit (Abeam Cat# ab6721, RRID:AB_955447) or anti-mouse (Abeam Cat# ab6789, RRID:AB_955439) secondary antibodies diluted 1:20000 for 30 minutes. Luminata Forte Western HRP substrate (Thermofisher) was added before imaging.

Western blots were imaged (LAS image) for 20 seconds, 1 minute, or automatic exposure and quantification was performed using ImageJ ³⁶. *MCM6* and TTK expression levels were normalized by dividing by their corresponding α-Tubulin expression levels and each drug treatment expression level was then normalized again by dividing by the mean normalized control expression level.

### Molecular Docking

Molecular docking analyses were performed to comprehend the molecular interaction mechanisms between the target proteins and their correspondingly repositioned drug candidates. 3D-coordinates of the *TTK* protein were retrieved from the protein data bank (PDB ID: 4C4J) while alpha-fold structure of *MCM6* was used ³⁷. Structures were optimized using quick prep tool of Molecular Operating Environment (MOE, V2019.01) suit to remove gaps, optimize angle, bond length, charges calculation and protonation of amino acids at physiological pH. The structures of the compounds were built using the MOE builder module and was charged and minimized by MMFF94x force field ³⁸. The standard default docking algorithm and scoring functions (triangle matcher algorithm, London dG and GBVI/WSA) were used. Hundred conformations were generated for each molecule that were analyzed based on clustering. Best suitable docked conformation was selected based on docking scores and interaction profile.

### Results

### Identification of Prognostic Genes

The univariate Cox survival analysis was performed based on the gene expression profiles and patients' OS in each cohort. As a result, 1,463 unfavorable genes whose high expression was associated with poor survival outcomes of patients and 166 favorable gene whose high expression was associated with good survival outcomes of patients in the TCGA cohort (p-value < 0.01) were identified. Similarly, 1,111 unfavorable genes and 4 favorable genes were identified in the Uppsala cohort (p-value < 0.01). The concordance of these prognostic genes from the two cohorts was evaluated and it was found that these two lists of unfavorable genes have a significant overlap (n=341, hypergeometric test, p-value < 1.0E-12). GO enrichment analysis showed that the 341 overlapped unfavorable genes were significantly enriched in chromosome organization and segregation, cell cycle, nuclear division, and DNA replication pathways. Similar pathways were observed from the KEGG enrichment analysis. A significant overlap was also observed between the two lists of favorable genes (n=3, hypergeometric test, p-value < 1.0E-12). Since there were only three overlapped favorable genes, *AMPD1, JCHAIN,* and *ZNF540,* their functions were examined individually. *AMPD1* encodes adenosine monophosphate deaminase which catalyzes AMP to IMP and it is a crucial enzyme in purine nucleotide and energy metabolism. It has been reported that *AMPD1* expression level is positively correlated to immune cell infiltration level in LUAD ³⁹. *JCHAIN* encodes immunoglobulin J-chain which is essential in the formation and stabilization of polymeric IgA and IgM structures. The transcription of *JCHAIN* in the lungs substantially decreases during tumorigenesis, which can be explained by the immunosuppressing effect of tumor cells ⁴⁰. *ZNF540* acts as a transcriptional repressor, and it is associated with CD8+ T cells infiltrating in LUAD ⁴¹.

### Identification of Functional Modules

Spearman correlation was performed to estimate the association between each two genes based on their gene expression levels in each cohort. The top 1% of the gene-to-gene links with the highest correlations coefficients were extracted to construct the gene co-expression network. Around 2,012,288 gene-to-gene links with correlation coefficients ranging from 0.55 to 1 were finally obtained in the gene co-expression network of the TCGA cohort. Further, a random walks-based algorithm, Walktrap, was used to identify the gene modules with high transitivity based on the topology of the CCN ³². The modules with more than 50 genes and clustering coefficients higher than 0.5 were used for further analysis. In the TCGA gene co-expression network, 14 modules (M1-M14, containing 57 to 3466 genes) were identified. The association of the modules with the 341 unfavorable genes were investigated based on concordance analysis (Table 1).

**Table 1.**

| Module name (TCGA cohort) | Number of genes in the module | Number of unfavorable signatures | Overlaps | P value of hypergeometric test |
|---|---|---|---|---|
| M1 | 3466 | 341 | 47 | 1,00E+00 |
| M2 | 831 | 341 | 1 | 1,00E+00 |
| M3 | 455 | 341 | 126 | < 1.0E-12 |
| M4 | 357 | 341 | 20 | 4,12E-04 |
| M5 | 286 | 341 | 0 | 1,00E+00 |
| M6 | 243 | 341 | 0 | 1,00E+00 |
| M7 | 227 | 341 | 0 | 1,00E+00 |
| M8 | 122 | 341 | 0 | 1,00E+00 |
| M9 | 89 | 341 | 0 | 1,00E+00 |
| M10 | 82 | 341 | 1 | 8,65E-01 |
| M11 | 80 | 341 | 0 | 1,00E+00 |
| M12 | 75 | 341 | 1 | 8,40E-01 |
| M13 | 70 | 341 | 3 | 2,37E-01 |
| M14 | 57 | 341 | 1 | 7,51E-01 |

It was observed that M3 (455 genes) and M4 (357 genes) had significant overlaps with these unfavorable genes in the TCGA cohort (n= 126 and 20, respectively, hypergeometric test, p < 0.01). Thus, these two modules were denoted as unfavorable modules. Functional enrichment showed that the genes involved in M3 were significantly enriched in the chromosome segregation, DNA replication and repair, nuclear division, and cell phase transition related pathways. The genes involved in the M4 were significantly enriched in the extracellular organization and angiogenesis pathways. Similar pathways were obtained by KEGG enrichment analysis.

Similarly, 1,979,922 gene-to-gene links with correlation coefficient ranging from 0.57 to 1 were obtained in the gene co-expression network of the Uppsala cohort and 12 modules (M1-M12, containing 51 to 2664 genes) were found. Among these modules, M4 (391 genes) and M7 (269 genes) had significant overlaps with the unfavorable genes (n= 19 and 99, respectively, hypergeometric test, p < 0.01, Table 2).

**Table**

| Module name (Uppsala cohort) | Number of genes in the module | Number of unfavorable signatures | Overlaps | P value of hypergeometric test |
|---|---|---|---|---|
| M1 | 2664 | 341 | 42 | 1,00E+00 |
| M2 | 1031 | 341 | 22 | 7,64E-01 |
| M3 | 579 | 341 | 1 | 1,00E+00 |
| M4 | 391 | 341 | 19 | 3,24E-03 |
| M5 | 372 | 341 | 7 | 8,02E-01 |
| M6 | 324 | 341 | 11 | 1,65E-01 |
| M7 | 269 | 341 | 99 | < 1.0E-12 |
| M8 | 264 | 341 | 1 | 9,99E-01 |
| M9 | 90 | 341 | 0 | 1,00E+00 |
| M10 | 58 | 341 | 2 | 4,12E-01 |
| M11 | 55 | 341 | 0 | 1,00E+00 |
| M12 | 51 | 341 | 0 | 1,00E+00 |

Functional enrichment analysis showed that the two modules exhibited an identical set of GO and KEGG pathways resulting from the TCGA cohort. Thus, it was inferred that the unfavorable modules identified from the TCGA and Uppsala cohorts were highly consistent. Based on the concordance analysis, it was observed that the TCGA M3 had a significant overlap with the Uppsala M7 (n= 242, hypergeometric test: p < 1.0E-12, Jaccard index = 0.5). Similar result was observed for the overlaps of TCGA M4 and Uppsala M4 (n=239, hypergeometric test: p < 1.0E-12, Jaccard index = 0.47). These results suggested that the unfavorable modules identified from the TCGA cohort were validated in the independent Uppsala cohort. In addition, a pairwise comparison was performed between the remaining modules identified in the two cohorts. Significant overlaps with high Jaccard indices were also found between TCGA M2 and Uppsala M3 which were enriched in adaptive immune system and cell differentiation pathways, TCGA M5 and Uppsala M8 which were enriched in angiogenesis and blood circulation pathways, TCGA M8 and Uppsala M9 which were enriched for extracellular transport and cilium assembly/organization pathways, and TCGA M11 and Uppsala M10 which were enriched in cytoplasmic translation, ribosome biogenesis, and p53 regulation pathways.

### Identification of Targetable Genes

To determine the hub genes that might drive the tumor development, topological analysis was performed to estimate the centrality of genes in these unfavorable modules. Degree, closeness and betweenness of genes were evaluated in each module. Then, the Spearman correlation of each centrality measurement between the pairwise matching of the unfavorable modules from the two cohorts was calculated. As a result, the correlation coefficients of degree, closeness and betweenness of genes were 0.86, 0.86 and 0.79 between TCGA M4 and Uppsala M4, and 0.8, 0.8 and 0.58 between TCGA M3 and Uppsala M7 (all p-values < 1.0E-12, Figure 1a and 1b). Further, the genes were ranked based on a descending order of each centrality measurement in each unfavorable module. The genes ranked within the top 20 in the matching unfavorable modules from the two cohorts were selected as the hub genes. A union set of genes identified based on all the centrality measurements were used as the final hub genes. Totally, 24 hub genes were identified. By including the three overlapped favorable genes, 27 hub genes were finally obtained. Further, the essential scores of these genes in 50 LUAD cell lines from were downloaded the DepMap data portal ³⁴. The essential score of a gene represents the essentiality of this gene for tumor cell proliferation and survival after knockout of this gene by CRISPR-Cas9 technology. A more negative score indicates worse survival of cells after the gene knockout. As shown in Figure 1c, nine hub genes, *KIF23, CDCA8, MAD2L1, MCM6, TPX2, NCAPH, CLSPN, TTK,* and *KIF4A,* whose essential scores in 50 LUAD cell lines were smaller than 0, were selected as the targetable genes (Figure 1c). These nine genes were all unfavorable for the patients' prognoses, whose hazard ratios were higher than 1 (Figure 1d).

### Drug Repositioning

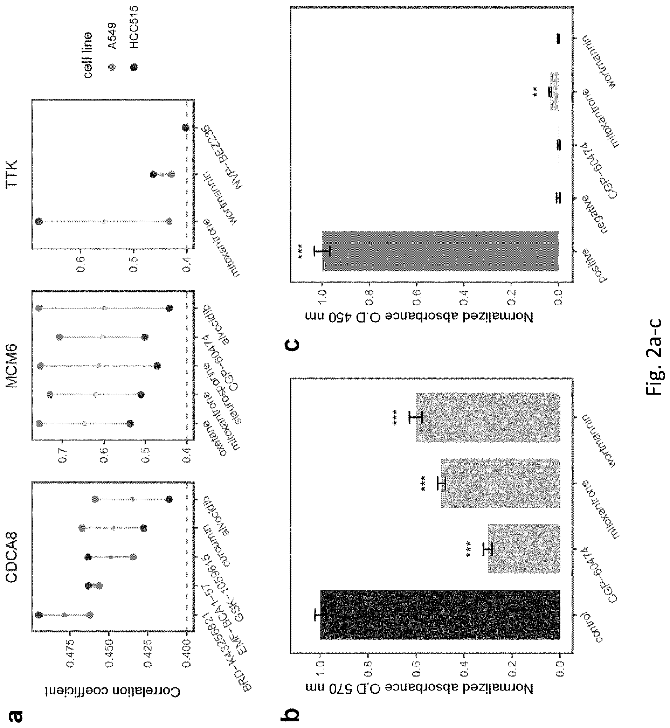

A previously proposed drug repositioning method was applied to identify promising drugs that can have an inhibitory effect on the expression of the targetable genes ^{15,24}. The algorithm is performed by comparing the similarity of molecular effect on tumor cells perturbed by a drug treatment and a gene knockout. This hypothesizes that a drug is considered to have an inhibitory effect on the expression of a target gene if this drug leads to a wide perturbation on the gene expression landscape in tumor cells which is similar to the effect of the knockout of this target gene (see details in the Method section). Among the nine targetable genes, only three genes *CDCA8, MCM6,* and *TTK* had the available transcriptomics signature profiles of two LUAD cell lines A549 and HCC515 that were perturbed by the drug treatment and the corresponding shRNA gene knockout from the LINCS database ⁴². In brief, the drug-shRNA matrix in which each element represents the similarity of the effect induced by a drug perturbagen and a shRNA gene knockdown perturbagen in each cell line was first constructed. To maximize the drug treated and genetic perturbed effects, the drug-shRNA matrix was simplified by extracting an optimal dosage/treatment duration for each drug and an optimal shRNA for each gene. Further, the two simplified drug-shRNA matrices from two cell lines were merged by extracting the same drugs. Finally, for each target gene, the top five drugs with the highest mean values of coefficients from two cell lines were selected as the potentially effective drugs. Meanwhile, the coefficients in each cell lines should be higher than 0.4. As shown in Figure 2a:
- BRD-K43256821, EMF-BCA1-57, GSK-1059615, curcumin, and alvocidib were identified as top candidate drugs for inhibiting *CDCA8;*
- oxetane, mitoxantrone, staurosporine, CGP-60474 and alvocidib were identified as top candidate drugs for inhibiting *MCM6;* and
- mitoxantrone, wortmannin and NVP-BEZ235 were identified as top candidate drugs for inhibiting *TTK.*

Among these drugs, only oxetane-containing drugs (e.g. taxol) and mitoxantrone are clinically used drugs, whereas the other are under medical investigation.

### Experimental Validation of Drug Efficacy

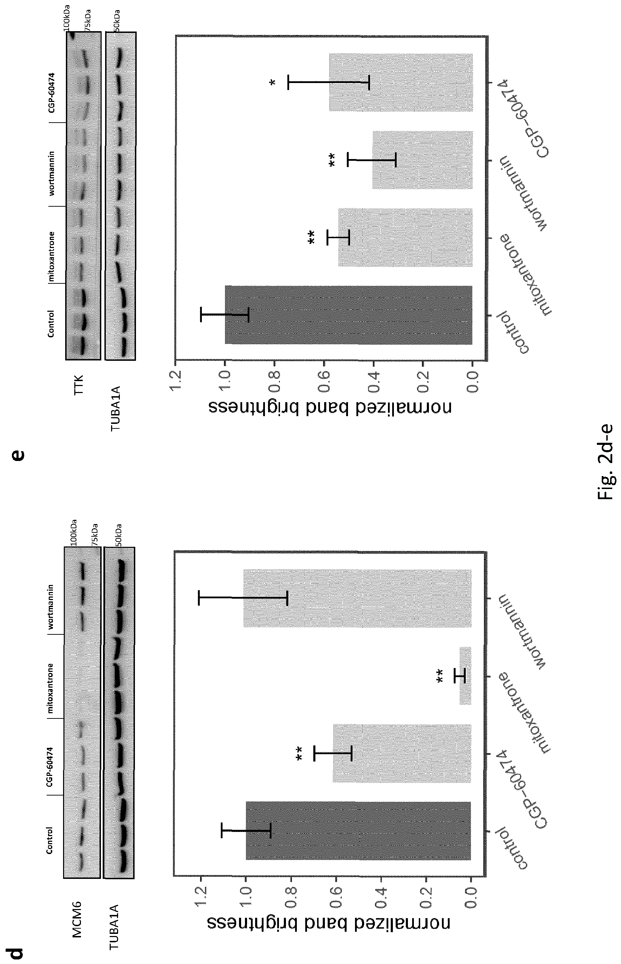

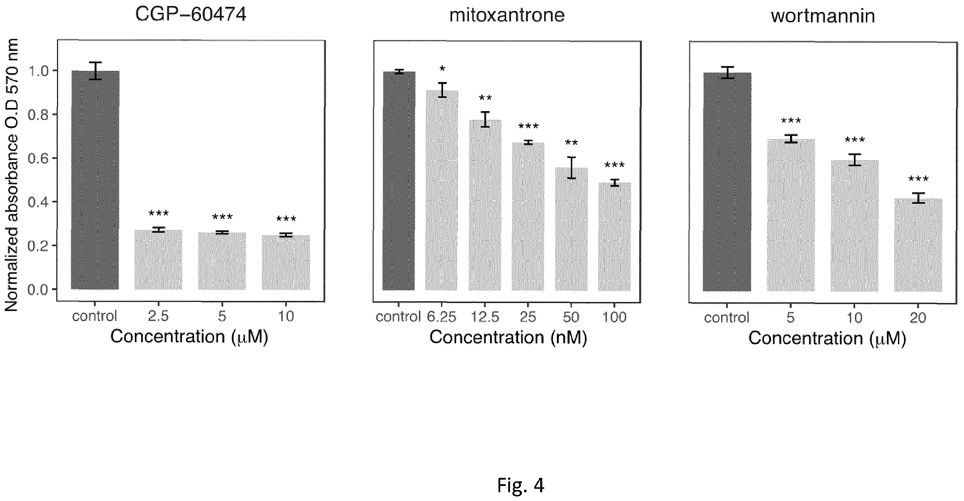

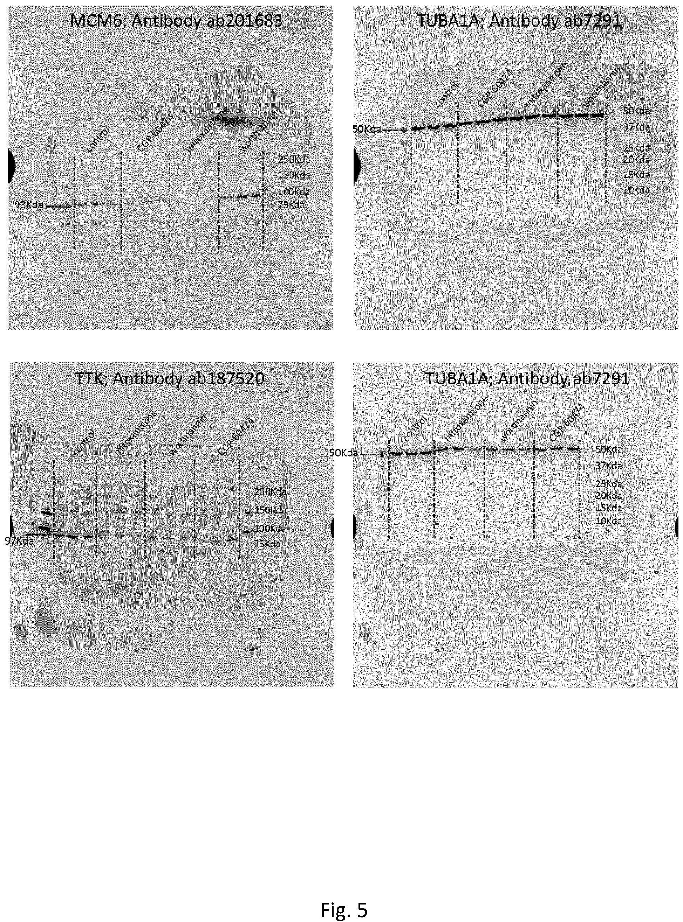

Among these repurposed drugs, only CGP-60474, mitoxantrone, and wortmannin could be obtained and hence tested in the *in vitro* model A549 cell line. First, dose dependent MTT assays were performed for each drug to select a proper concentration which could reduce the cell viability by 50%. This concentration shows moderate toxicity and is suitable for further experiments to study the drug effect in the cell line. As a result, 10µM for CGP-60474, 10µM for wortmannin and 100nM for mitoxantrone were determined (Figure 4). Further, the MTT assay was used for cell viability and the LDH assay was used for cytotoxicity. In the MTT assay, cell viability was decreased to 29.1% ±0.7 by CGP-60474, 52.2% ±1.4 by mitoxantrone and, 62.3% ±2.4 by wortmannin (Figure 2b). Interestingly, LDH assays showed that 10µM CGP-60474 and 10µM wortmannin did not induce necrotic cell death, and1 00nM of mitoxantrone showed only 3.45% ±0.4 necrotic cell death compared to the lysis positive control (100%) and negative untreated group (0%) (Figure 2c). Taken together, cell viability and cytotoxicity assay results indicated that these drugs decreased the cell viability mainly by inhibiting the cell proliferation rather than inducing necrotic cell death procedure. To investigate whether the repurposed drugs could inhibit their corresponding genes (that they were repurposed for), the protein expression levels of these target genes were evaluated in the A549 cell lines after drug treatment. Western blots showed that CGP-60474 and mitoxantrone significantly reduced the protein level of *MCM6* (Figures 2d and 5). Especially, mitoxantrone showed an extremely effective suppression on *MCM6,* with 95% decrease of protein expression (Figure 2d). In addition, mitoxantrone and wortmannin significantly inhibited the protein level of *TTK* by around 50% (Figures 2e and 5). Notably, CGP-60474, which was predicted to inhibit *MCM6,* also showed an inhibitory effect on *TTK.* These results suggested that the repurposed drugs effectively targeted the corresponding genes and reduced the tumor cell proliferation.

### Molecular Docking

The molecular docking analyses were performed to gain a deep insight of the binding mode between the validated drugs (mitoxantrone, CGP-60474, and wortmannin) and the target proteins (*TTK* and *MCM6*)*.* The predicted docking scores for the binding interactions of mitoxantrone, CGP-60474 and wortmannin against *TTK* were -9.39, -8.18 and -7.28 (kcal/mol), respectively, representing moderate to strong interactions (Table 3).

**Table 3.**

| Compound | Docking scores (Kcal/mol) | Hydrogen Bonds interactions | | | Hydrophobic Interactions |
|---|---|---|---|---|---|
| | | Donor | Acceptor | Distance | |
| Mitoxantrone | -9.3935 | OH | COO- of Glu603 | 2.11 | Ile531 |
| | | NH | COO-of Gly605 | 3.13 | Val539 |
| | | | | | Ala551 |
| | | NH of Gly605 | OH | 2.39 | Asp608 |
| | | | | | Leu654 |
| | | | COO- of Ser611 | 2.69 | Ile663 |
| | | OH | | | |
| | | | COO- of Ala651 | 1.9 | |
| | | NH | | | |
| | | | N3 | 3.2 | |
| | | NH of Gln670 | O3 | 2.82 | |
| | | NH of Met671 | | | |
| CGP-60474 | -8.1757 | NH | COO- of Gly605 | 2.36 | Ile531 |
| | | | | | Val539 |
| | | NH of Gly605 | N of pyrimidine ring | 2.27 | Ala551 |
| | | | | | Asp608 |
| | | | | | Leu654 |
| | | OH | COO- of Asp608 | 2.71 | Ile663 |
| Wortmannin | -7.2825 | NH of Lys553 | =CO | 2.39 | Ile531 |
| | | | | | Val539 |
| | | | =CO of tetrahydropyran-2-one | 3.18 | Ala551 |
| | | NH of Asp608, | | | Leu654 |
| | | | | | Ile663 |
| | | | O2 of furan ring | 3.36 | Asp664 |
| | | NH of Gln670 | =CO of tetrahydropyran-2-one | 2.97 | |
| | | | | | |
| | | NH Asp674 | | | |
| | | | | | |

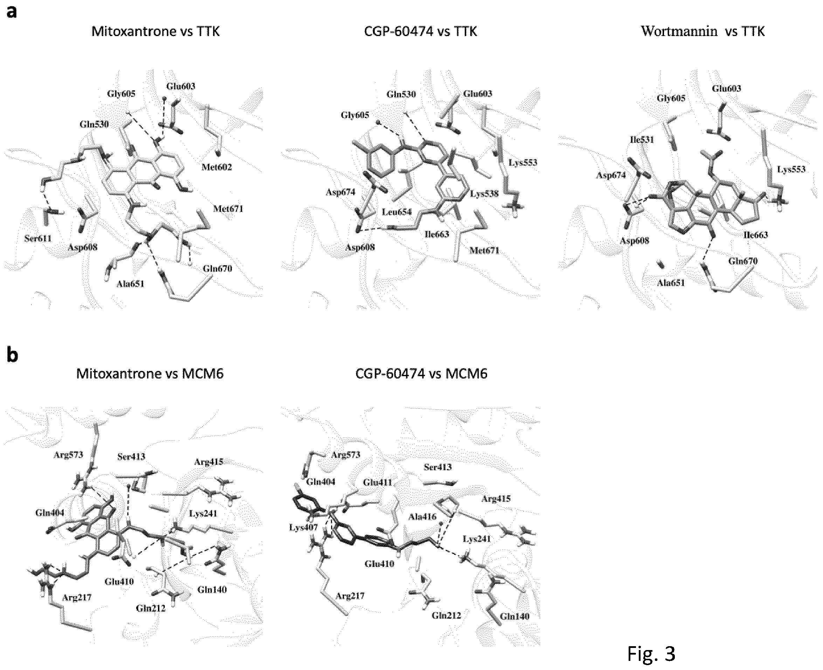

Further, investigation revealed that the comparative potential of the compounds mostly due to the hydrophilic interactions (Glu603, Gly605, Ser611, Ala651, Gln671 and Met674) (Figure 3a), while the aromatic ring of the compounds confers stability by forming hydrophobic interactions with the active site residues (Ile531, Val539, Ala551, Leu654, Ile663 and Asp664). The predicted docking scores for the binding interactions of mitoxantrone and CGP-60474 against *MCM6* were - 7.54 and -6.37 (kcal/mol), respectively, representing moderate interactions (Table 4). Both drug candidates interacted with crucial residues of the protein by establishing hydrophilic (Gln212, Ala213, Arg217, Arg217, Lys241, Ser413 and Arg573) and hydrophobic (Gln212, Ala213, Glu410 and Glu411) interaction (Figure 3b).

**Table 4.**

| Compounds | Docking scores (Kcal/mol) | Hydrogen Bonds interactions | | | Hydrophobic Interactions |
|---|---|---|---|---|---|
| | | Donor | Acceptor | Distance | |
| CGP-60474 | -6.3695 | NH of Arg₂₁₇ | Npl of ligand | 3.08 | Gln₂₁₂ |
| | | | Npl of ligand | 2.42 | Ala₂₁₃ |
| | | NH of Arg₂₁₇ | O₃ of ligand | 2.93 | Glu₄₁₀ |
| | | | Npl of ligand | 2.90 | Glu₄₁₁ |
| | | N₃+ of Lys₂₄₁ | COO- of Glu₄₁₀ | 3.0 | |
| | | | | 2.16 | |
| | | OH Glu₄₁₀ | COO- of Glu₄₁₁ | 3.45 | |
| | | Npl of ligand | | 2.07 | |
| | | | O₂ of ligand | | |
| | | Npl of ligand | COO- of Ala₄₁₆ | | |
| | | | | | |
| | | NH of Ala₄₁₆ | | | |
| | | | | | |
| | | OH of ligand | | | |
| | | | | | |
| Mitoxantrone | -7.5429 | OH of ligand | COO- of Gln₁₄₀ | 2.0 | Gln₂₁₂ |
| | | | | 2.63 | Ala₂₁₃ |
| | | NH of Gln₂₁₂ | O₂ of ligand | 3.25 | Glu₄₁₀ |
| | | | N₃ of ligand | 2.26 | Glu₄₁₁ |
| | | NH of Ala₂₁₃ | N₃ of ligand | 2.99 | |
| | | | N₃ of ligand | 1.91 | |
| | | NH of Arg₂₁₇ | N₃ of ligand | 2.14 | |
| | | | COO- of Ser₄₁₃ | 3.53 | |
| | | NH of Arg₂₁₇ | | | |
| | | | =CO of ligand | | |
| | | NH of Lys₂₄₁ | | | |
| | | | | | |
| | | NH of ligand | | | |
| | | | | | |
| | | NH of Arg₅₇₃ | | | |

### REFERENCES

1. Sung H, Ferlay J, Siegel RL, et al. Global Cancer Statistics 2020: GLOBOCAN Estimates of Incidence and Mortality Worldwide for 36 Cancers in 185 Countries. CA Cancer J Clin 2021; 71(3): 209-49.
2. Behrend SJ, Giotopoulou GA, Spella M, Stathopoulos GT. A role for club cells in smoking-associated lung adenocarcinoma. Eur Respir Rev 2021; 30(162).
3. Ettinger DS, Wood DE, Aisner DL, et al. Non-Small Cell Lung Cancer, Version 3.2022, NCCN Clinical Practice Guidelines in Oncology. J Natl Compr Canc Netw 2022; 20(5): 497-530.
4. Hirsch FR, Scagliotti GV, Mulshine JL, et al. Lung cancer: current therapies and new targeted treatments. Lancet 2017; 389(10066): 299-311.
5. Melocchi V, Dama E, Mazzarelli F, et al. Aggressive early-stage lung adenocarcinoma is characterized by epithelial cell plasticity with acquirement of stem-like traits and immune evasion phenotype. Oncogene 2021; 40(31): 4980-91.
6. Kadara H, Behrens C, Yuan P, et al. A five-gene and corresponding protein signature for stage-I lung adenocarcinoma prognosis. Clin Cancer Res 2011; 17(6): 1490-501.
7. Feng Z, Zhang Y, He M, et al. AMICA1 is a diagnostic and prognostic biomarker and induces immune cells infiltration by activating cGAS-STING signaling in lung adenocarcinoma. Cancer Cell Int 2022; 22(1): 111.
8. He M, Han Y, Cai C, et al. CLEC10A is a prognostic biomarker and correlated with clinical pathologic features and immune infiltrates in lung adenocarcinoma. J Cell Mol Med 2021; 25(7): 3391-9.
9. Liu XS, Zhou LM, Yuan LL, et al. NPM1 Is a Prognostic Biomarker Involved in Immune Infiltration of Lung Adenocarcinoma and Associated With m6A Modification and Glycolysis. Front Immunol 2021; 12: 724741.
10. He Y, Liu R, Yang M, et al. Identification of VWF as a Novel Biomarker in Lung Adenocarcinoma by Comprehensive Analysis. Front Oncol 2021; 11: 639600.
11. Lu M, Fan X, Liao W, et al. Identification of significant genes as prognostic markers and potential tumor suppressors in lung adenocarcinoma via bioinformatical analysis. BMC Cancer 2021; 21(1): 616.
12. Bian T, Zheng M, Jiang D, et al. Prognostic biomarker TUBA1C is correlated to immune cell infiltration in the tumor microenvironment of lung adenocarcinoma. Cancer Cell Int 2021; 21(1): 144.
13. Jiawei Z, Min M, Yingru X, et al. Identification of Key Genes in Lung Adenocarcinoma and Establishment of Prognostic Mode. Front Mol Biosci 2020; 7: 561456.
14. Pushpakom S, Iorio F, Eyers PA, et al. Drug repurposing: progress, challenges and recommendations. Nat Rev Drug Discov 2019; 18(1): 41-58.
15. Li X, Shong K, Kim W, et al. Prediction of drug candidates for clear cell renal cell carcinoma using a systems biology-based drug repositioning approach. EBioMedicine 2022; 78: 103963.
16. Lamb J, Crawford ED, Peck D, et al. The Connectivity Map: using gene-expression signatures to connect small molecules, genes, and disease. Science 2006; 313(5795): 1929-35.
17. Iskar M, Campillos M, Kuhn M, Jensen LJ, van Noort V, Bork P. Drug-induced regulation of target expression. PLoS ComputBiol 2010; 6(9).
18. Nagaraj AB, Wang QQ, Joseph P, et al. Using a novel computational drug-repositioning approach (DrugPredict) to rapidly identify potent drug candidates for cancer treatment. Oncogene 2018; 37(3): 403-14.
19. Pillaiyar T, Meenakshisundaram S, Manickam M, Sankaranarayanan M. A medicinal chemistry perspective of drug repositioning: Recent advances and challenges in drug discovery. Eur JMed Chem 2020; 195: 112275.
20. Jin G, Wong ST. Toward better drug repositioning: prioritizing and integrating existing methods into efficient pipelines. Drug Discov Today 2014; 19(5): 637-44.
21. Huang RX, Siriwanna D, Cho WC, et al. Lung adenocarcinoma-related target gene prediction and drug repositioning. Front Pharmacol 2022; 13: 936758.
22. De Bastiani MA, Klamt F. Integrated transcriptomics reveals master regulators of lung adenocarcinoma and novel repositioning of drug candidates. Cancer Med 2019; 8(15): 6717-29.
23. Kwon OS, Lee H, Kong HJ, et al. Connectivity map-based drug repositioning of bortezomib to reverse the metastatic effect of GALNT14 in lung cancer. Oncogene 2020; 39(23): 4567-80.
24. Zhang C, Shi M, Kim W, et al. Discovery of therapeutic agents targeting PKLR for NAFLD using drug repositioning. EBioMedicine 2022; 83: 104214.
25. Yuan M, Shong K, Li X, et al. A Gene Co-Expression Network-Based Drug Repositioning Approach Identifies Candidates for Treatment of Hepatocellular Carcinoma. Cancers (Basel) 2022; 14(6).
26. Cancer Genome Atlas Research N. Comprehensive molecular profiling of lung adenocarcinoma. Nature 2014; 511(7511): 543-50.
27. Mezheyeuski A, Bergsland CH, Backman M, et al. Multispectral imaging for quantitative and compartment-specific immune infiltrates reveals distinct immune profiles that classify lung cancer patients. JPathol 2018; 244(4): 421-31.
28. Goldmann T, Marwitz S, Nitschkowski D, et al. PD-L1 amplification is associated with an immune cell rich phenotype in squamous cell cancer of the lung. Cancer Immunol Immunother 2021; 70(9): 2577-87.
29. Bray NL, Pimentel H, Melsted P, Pachter L. Erratum: Near-optimal probabilistic RNA-seq quantification. NatBiotechnol 2016; 34(8): 888.
30. Therneau T, Lumley T. R survival package. R Core Team 2013.
31. Yu G, Wang LG, Han Y, He QY. clusterProfiler: an R package for comparing biological themes among gene clusters. OMICS 2012; 16(5): 284-7.
32. Pons P, Latapy M. Computing Communities in Large Networks Using Random Walks. International Symposium on Computer and Information Sciences 2005; 3733: 284-93.
33. Nascimento M, Carvalho A. A graph clustering algorithm based on a clustering coefficient for weighted graphs. Journal of the Brazilian Computer Society 2011; 17: 19.
34. Meyers RM, Bryan JG, McFarland JM, et al. Computational correction of copy number effect improves specificity of CRISPR-Cas9 essentiality screens in cancer cells. Nat Genet 2017; 49(12): 1779-84.
35. Subramanian A, Narayan R, Corsello SM, et al. A Next Generation Connectivity Map: L1000 Platform and the First 1,000,000 Profiles. Cell 2017; 171(6): 1437-52 e17.
36. Schneider CA, Rasband WS, Eliceiri KW. NIH Image to ImageJ: 25 years of image analysis. Nat Methods 2012; 9(7): 671-5.
37. Naud S, Westwood IM, Faisal A, et al. Structure-based design of orally bioavailable 1H-pyrrolo[3,2-c]pyridine inhibitors of mitotic kinase monopolar spindle 1 (MPS1). J Med Chem 2013; 56(24): 10045-65.
38. Halgren TA. Merck molecular force field. I. Basis, form, scope, parameterization, and performance of MMFF94. Journal of computational chemistry 1996; 17: 490.
39. Xu ZY, Zhao M, Chen W, et al. Analysis of prognostic genes in the tumor microenvironment of lung adenocarcinoma. PeerJ 2020; 8: e9530.
40. Slizhikova DK, Zinov'eva MV, Kuz'min DV, et al. [Decrease in expression of human J-chain in lung squamous cell cancer and adenocarcinoma]. Mol Biol (Mosk) 2007; 41(4): 659-65.
41. Zhang M, Ma J, Guo Q, Ding S, Wang Y, Pu H. CD8(+) T Cell-Associated Gene Signature Correlates With Prognosis Risk and Immunotherapy Response in Patients With Lung Adenocarcinoma. Front Immunol 2022; 13: 806877.
42. Stathias V, Turner J, Koleti A, et al. LINCS Data Portal 2.0: next generation access point for perturbation-response signatures. Nucleic Acids Res 2020; 48(D1): D431-D9.
43. Hayama S, Daigo Y, Yamabuki T, et al. Phosphorylation and activation of cell division cycle associated 8 by aurora kinase B plays a significant role in human lung carcinogenesis. Cancer Res 2007; 67(9): 4113-22.
44. Lauze E, Stoelcker B, Luca FC, Weiss E, Schutz AR, Winey M. Yeast spindle pole body duplication gene MPS1 encodes an essential dual specificity protein kinase. EMBO J 1995; 14(8): 1655-63.
45. Zheng L, Chen Z, Kawakami M, et al. Tyrosine Threonine Kinase Inhibition Eliminates Lung Cancers by Augmenting Apoptosis and Polyploidy. Mol Cancer Ther 2019; 18(10): 1775-86.
46. Furet P, Zimmermann J, Capraro HG, Meyer T, Imbach P. Structure-based design of potent CDK1 inhibitors derived from olomoucine. J Comput Aided Mol Des 2000; 14(5): 403-9.
47. Hogg D, Guidos C, Bailey D, et al. Cell cycle dependent regulation of the protein kinase TTK. Oncogene 1994; 9(1): 89-96.
48. Bashir T, Pagano M. Cdk1: the dominant sibling of Cdk2. Nat Cell Biol 2005; 7(8): 779-81.
49. Evison BJ, Sleebs BE, Watson KG, Phillips DR, Cutts SM. Mitoxantrone, More than Just Another Topoisomerase II Poison. Med Res Rev 2016; 36(2): 248-99.
50. Suga J, Saijo N, Shinkai T, et al. Phase II study of mitoxantrone in patients with non-small cell lung cancer. Jpn J Clin Oncol 1986; 16(2): 147-51.
51. Feun LG, Savaraj N, Solomon J, Liebmann A, Hurley J. Phase II trial of mitoxantrone and cisplatin in advanced non-small-cell lung cancer. Am J Clin Oncol 1996; 19(2): 190-2.
52. Powis G, Bonjouklian R, Berggren MM, et al. Wortmannin, a potent and selective inhibitor of phosphatidylinositol-3-kinase. Cancer Res 1994; 54(9): 2419-23.
53. Boehle AS, Kurdow R, Boenicke L, et al. Wortmannin inhibits growth of human non-small-cell lung cancer in vitro and in vivo. Langenbecks Arch Surg 2002; 387(5-6): 234-9.
54. Zhang Y, Bao C, Mu Q, et al. Reversal of cisplatin resistance by inhibiting PI3K/Akt signal pathway in human lung cancer cells. Neoplasma 2016; 63(3): 362-70.

## Claims

1. CGP-60474 or a pharmaceutically acceptable salt thereof for use in a method of prevention or treatment of lung adenocarcinoma.

2. CGP-60474 or a pharmaceutically acceptable salt thereof for use according to claim 1, wherein said method further comprises administration of a drug selected from the group consisting of BRD-K43256821, EMF-BCA1-57, GSK-1059615, curcumin and alvocidib.

3. CGP-60474 or a pharmaceutically acceptable salt thereof for use according to claim 1 or 2, wherein said method further comprises administration of a drug selected from the group consisting of mitoxantrone, wortmannin and NVP-BEZ235.

4. CGP-60474 or a pharmaceutically acceptable salt thereof for use according to any one of the preceding claims, wherein said lung adenocarcinoma expresses *MCM6.*

5. CGP-60474 or a pharmaceutically acceptable salt thereof for use according to any one of the preceding claims, wherein said lung adenocarcinoma overexpresses *MCM6* as compared to an average expression value obtained from measurements in healthy tissue.

6. Mitoxantrone or a pharmaceutically acceptable salt thereof for use in a method of treatment of stage 0 or I lung adenocarcinoma.

7. Mitoxantrone or a pharmaceutically acceptable salt thereof for use according to claim 6, wherein said method further comprises administration of a drug selected from the group consisting of BRD-K43256821, EMF-BCA1-57, GSK-1059615, curcumin and alvocidib.

8. Mitoxantrone or a pharmaceutically acceptable salt thereof for use according to claim 6 or 7, wherein said method further comprises administration of a drug selected from the group consisting of oxetane, staurosporine, CGP-60474 and alvocidib.

9. Mitoxantrone or a pharmaceutically acceptable salt thereof for use according to any one of claims 6-8, wherein said lung adenocarcinoma expresses TTK.

10. Mitoxantrone or a pharmaceutically acceptable salt thereof for use according to any one of claims 6-9, wherein said lung adenocarcinoma overexpresses TTK as compared to an average expression value obtained from measurements in healthy tissue.
